# EUROPÄISCHE PATENTSCHRIFT

(11) **EP 1 389 088 B1**
(45) Veröffentlichungstag und Bekanntmachung des Hinweises auf die Patenterteilung: **11.11.2009**
(21) Anmeldenummer: 02771642.2
(22) Anmeldetag: 11.05.2002
(51) Int. Cl.: A61Q 17/00, A61K 8/06, A61K 8/64

(54) **LIPOPROTEIN-CREMES MIT UV-FILTERN**
LIPOPROTEIN CREAMS WITH UV FILTERS
CREMES LIPOPROTEIQUES CONTENANT DES FILTRES UV

(30) Priorität: 22.05.2001 DE 10124914
(43) Veröffentlichungstag der Anmeldung: 18.02.2004
(73) Patentinhaber: Henkel AG & Co. KGaA, 40589 Düsseldorf (DE)
(72) Erfinder: YÜCEL, Sevda, 41470 Neuss (DE); CALAS, Marie, 08026 Barcelona (ES); WALDMANN-LAUE, Marianne, 40789 Monheim (DE)
(86) Internationale Anmeldenummer: PCT/EP2002/005200
(87) Internationale Veröffentlichungsnummer: WO 2002/094209

(56) Entgegenhaltungen:
- WO-A-94/21222
- WO-A-99/36051
- FR-A- 2 771 293
- US-A- 5 607 666
- US-A- 5 989 529

## Beschreibung

Die Erfindung betrifft kosmetische und pharmazeutische Zusammensetzungen mit UV-Schutz in Form von Öl-in-Wasser-Emulsionen polarer Ölkomponenten, die als Emulgatoren gelöste pflanzliche Proteine enthalten. Solche Emulsionen lassen sich ohne die Verwendung üblicher ionischer oder hydrophiler nichtionischer Emulgatoren formulieren. Die erfindungsgemäßen Zusammensetzungen weisen eine besonders hohe Hautpflegewirkung auf und beugen dem Risiko einer allergischen Reaktion, wie z. B. der sogenannten Mallorca-Akne, vor. Weiterhin sind in der Kosmetik pflanzliche Proteine gegenüber tierischen Proteinen zunehmend bevorzugt.

Der Einsatz von Sojaprotein in Kombination mit üblichen ethoxylierten Emulgatoren und Phospholipiden zur Herstellung von Lipoprotein-Emulsionen, z.B. für die Nahrungsmittel-und Backwaren-Industrie ist aus US 4,360,537 bekannt. Auch für kosmetische Emulsionen sind Proteine als Emulgatoren beschrieben worden. So ist z. B. aus WO 94/21222 A1 sowie aus WO 95/11663 A1 bekannt, proteinhaltige Mehle, zum Beispiel gemahlene Samen von Leguminosen oder Getreide, als partikuläre Emulgatoren für Sonnenschutz-Lotionen zu verwenden. Allerdings weisen solche Emulsionen, wenn sie nicht zusätzlich hydrophile Emulgatoren enthalten, nur eine sehr begrenzte Stabilität auf.
Die WO 90/05521 A1 sowie die WO 94/18944 offenbaren kosmetische Zusammensetzungen, die gelöste pflanzliche Proteine enthalten. Die offenbarten Zusammensetzungen enthalten ionische, ethoxylierte oder andere Emulgatoren und Tenside mit einem HLB-Wert oberhalb von 6,5. Es gibt keinen Hinweis auf eine Kombination mit UV-Filtem.
Die Offenlegungsschrift JP 58-65208 (Asahi Denka Kogyo K.K.) beschreibt kosmetische Emulsionen, die als Emulgator eine Mischung aus Proteinen oder Proteinbausteinen und Polyhydroxy-Verbindungen oder deren wässrigen Lösungen enthalten. Weder polare noch unpolare Ölkomponenten sind als bevorzugt offenbart.

Die US-Patentschrift US 5,607,666 beschreibt kosmetische Zusammensetzungen in Pulverform, die sich mit Wasser oder anderen wässrigen Zubereitungen rehydratisieren lassen. Die durch Wasserzugabe erhaltenen Öl-in-Wasser-Emulsionen enthalten als Emulgator und Strukturgeber die Kombination aus einem Hydrocolloid, ausgewählt aus Xanthan Gum, Carboxymethylcellutose und Maisstärke, und einem Biopolymer, ausgewählt aus Natriumkaseinat, Kasein, Sojaprotein und Sojaproteinhydrolysat.
Die Druckschrift WO 99/36051 beschreibt kosmetische Proteinemulsionen, gibt aber keinen Hinweis auf die Herstellung stabiler Emulsionen, die schwer lösliche organische UV-Filter enthalten.

Die vorliegende Erfindung beruht auf der Beobachtung, dass bestimmte pflanzliche Proteine in wässriger Lösung nicht nur die Oberflächenspannung des Wassers, d. h. die Grenzflächenspannung Wasser/Luft, senken, sondern auch die Grenzflächenspannung von Wasser zu einer angrenzenden Ölphase. Wenn die Ölphase eine gewisse Polarität aufweist, lassen sich mit solchen Proteinpräparaten sehr stabile, UV-Filter enthaltende Öl-in-Wasser-Emulsionen herstellen. Bevorzugt sind solche Öle und Ölmischungen, die zu Wasser eine Grenzflächenspannung Wasser/Öl unterhalb von 30 mN/m (25° C) aufweisen. Eine Aufgabe der vorliegenden Erfindung war es, geeignete Proteinpräparate, darauf abgestimmte Ölkomponenten und UV-Filter zu finden, die aufgrund ihrer Grenzflächeneigenschaften auch ohne Mitwirkung üblicher O/W-Emulgatoren die Herstellung stabiler Emulsionen mit UV-Schutz möglich machen. Die dafür geeigneten pflanzlichen Proteine und Proteinhydrolysate werden aufgrund ihrer lipophilen Eigenschaften im folgenden auch als Lipoproteine bezeichnet. Neben der emulgierenden Wirkung weisen die wasserlösliche Proteine eine kosmetisch günstige Hautwirkung auf, indem sie zur Bildung nichtfettender Proteinfilme auf der Haut mit gutem Wasserrückhaltevermögen führen. Eine weitere Aufgabe der Erfindung bestand darin, Sonnenschutzzusammensetzungen zur Verfügung zu stellen, die frei sind von ionischen oder hydrophilen, insbesondere alkoxylierten nichtionischen Emulgatoren.

Gegenstand der Erfindung sind kosmetische und pharmazeutische Zusammensetzungen mit UV-Schutz in Form einer Öl-in-Wasser-Emulsion, die mindestens ein gelöstes pflanzliches Protein oder Proteinhydrolysat, dessen wässrige Lösung in einer Konzentration von 1 Gew.-% gegenüber der Ölkomponente eine Grenzflächenspannung bei 25° C (γ²) aufweist, die niedriger ist als die Grenzflächenspannung γ¹ zwischen Ölkomponenten und Wasser, mindestens eine polare Ölkomponente, ausgenommen Olivenöl, mindestens einen organischen oder anorganischen UV-Filter sowie Mischungen hiervon enthalten und die frei von ionischen Emulgatoren, alkoxylierten Emulgatoren und Emulgatoren mit einem HLB-Wert über 6,5 sind.
Das erfindungsgemäß verwendete pflanzliche Protein oder Proteinhydrolysat bewirkt in wässriger Lösung gegenüber der polaren Ölkomponente eine Erniedrigung der Grenzflächenspannung Öl/Wasser und dient aufgrund dieser Grenzflächenaktivität als Emulgator. Bevorzugt sind solche pflanzlichen Proteine oder Proteinhydrolysate enthalten, die in 1 Gew.-%iger Lösung bei 25° C die Grenzflächenspannung des Wassers zur Ölkomponente um einen Betrag (γ¹ - γ²) von wenigstens 2 mN/m senken.
Geeignete Proteine und Proteinhydrolysate, die diese Anforderungen im Zusammenwirken mit den erfindungsgemäß geeigneten polaren Ölkomponenten erfüllen, stammen aus Pflanzensamen. Erfindungsgemäß geeignet sind beispielsweise Mandel-, Haselnuß-und Baumwollsamenprotein sowie Proteine und Proteinhydrolysate aus den Samen von Hibiscus esculentus (Okra). Weitere geeignete Proteine und Proteinhydrolysate werden aus Leguminosen, z. B. aus Erbsen, und aus Getreide, insbesondere aus Hafer, Weizen und Soja, gewonnen.
Bevorzugt sind Proteine und Proteinhydrolysate aus Erbsen, Hafer, Weizen und den Samen von Hibiscus esculentus. Ganz besonders bevorzugte Proteine und Proteinhydrolysate stammen aus den Samen von Hibiscus esculentus und aus Erbsen.

Isolations- und Reinigungsverfahren für pflanzliche Proteine sind vielfach in der Patentliteratur beschrieben. So ist z.B. aus EP 371 601 A2 ein Verfahren zur Isolierung einer tensidartigen Proteinfraktion aus Hafer bekannt.

Üblicherweise beträgt der Gehalt an wasserlöslichem bzw. in Wasser gelöstem Protein mindestens 0,05 Gew.-% und maximal 5 Gew.-%, bezogen auf die Gesamtzusammensetzung.
Bevorzugt werden 0,05 - 0,5 Gew.-% Aktivsubstanz des Proteins oder Proteinhydrolysats, besonders bevorzugt 0,1 - 0,3 Gew.-%, jeweils bezogen auf die erfindungsgemäße Zusammensetzung, eingesetzt.
Die erfindungsgemäß bevorzugten Proteine oder Proteinhydrolysate enthalten Globuline mit einem Molgewicht von mehr als 30.000 Dalton in Anteilen von 50 - 90 %, bevorzugt 60 - 85 % und Albumine mit einem Molgewicht unter 30.000 Dalton in Anteilen von 10 - 50%, bevorzugt 15 - 40 %, bezogen auf den Proteingehalt.
Ein besonders bevorzugtes Erbsenprotein ist als Produkt "V-Protein liquid" von Cosmetochem erhältlich und enthält 18 % Albumine (Molgewicht 15.000 bis 30.000 Dalton) sowie 82 % Globuline (Molgewicht 150.000 bis 300.000 Dalton). Ein besonders bevorzugtes Protein aus den Samen von Hibiscus esculentus ist als Produkt Hibiscin^{®} HP LS 9198 von Laboratoires Sérobiologiques erhältlich, das ein durchschnittliches Molgewicht von etwa 500.000 Dalton und folgende Zusammensetzung der Molgewichtsfraktionen aufweist:
- Größer als 500.000 Dalton : 48,4 %
- 100.000 bis 500.000 Dalton: 4,8 %
- 30.000 bis 100.000 Dalton: 8,5 %
- 5000 bis 30.000 Dalton: 22,0 %
- kleiner als 5000 Dalton: 16,3 %.

Hibiscin^{®} HP LS 9198 hat gegenüber dem V-Protein liquid den Vorteil, dass es in geringeren Mengen eingesetzt werden kann.

In einer bevorzugten Ausführungsform enthalten die erfindungsgemäßen Zusammensetzungen, bezogen auf die Gesamtzusammensetzung, 30 bis 75 Gew.-% Wasser, besonders bevorzugt 35 bis 65 Gew.-% Wasser.

Die polaren Ölkomponenten sind ausgewählt aus den Estern von gesättigten und ungesättigten, linearen und verzweigten C₃-C₂₂-Fettsäuren und den Dimeren ungesättigter C₁₂-C₂₂-Fettsäuren (Dimerfettsäuren) mit a) einwertigen linearen, verzweigten und cyclischen C₂-C₁₈-Alkanolen, b) mehrwertigen linearen und verzweigten C₂-C₆-Alkanolen, c) Polyglycerinen der Formel CH₂OH-CHOH-CH₂[-O-CH₂-CHOH-CH₂]ₙ-O-CH₂-CHOH-CH₂OH mit n = 0 - 8, und d) den Dicarbonsäureestern von linearen und verzweigten C₂-C₁₀-Alkanolen, den Benzoesäureestern von linearen und verzweigten C₁₀-C₂₀-Alkanolen, den C₁₂-C₂₂-Alkanolestem ein- und mehrwertiger C₂-C₇-Hydroxycarbonsäuren, die aromatisch sein können, sowie Mischungen dieser Komponenten, ausgenommen Olivenöl.
Beispiele für erfindungsgemäß besonders geeignete Fettsäureester einwertiger linearer oder verzweigter C₂-C₁₈-Alkanole (Typ a) sind Octylethylhexanoat, Isopropylpalmitat, Hexyllaurat, Isopropylmyristat, ein Gemisch der Ester von Caprylsäure und Caprinsäure mit C₁₂-C₁₈-Fettalkoholen, das als Handelsprodukt Cetiol^{®} LC (Cognis) erhältlich ist, C₁₂-C₁₅-Alkyloctanoat, Isodecylneopentanoat, Isononylisononanoat, 2-Ethylhexylpalmitat (z. B. Cegesoft^{®} C 24 von Cognis), 2-Ethylhexylstearat, Cetearylisononanoat (Mischung aus Cetylisononanoat und Stearylisononanoat, z. B. Cetiol^{®} SN (Cognis)), Cetearyloctanoat, Ethylstearat, Isopropylstearat, Butylstearat und Myristylpropionat. Weiterhin geeignet sind die Monoester des Isosorbid (1,4:3,6-Dianhydro-D-glucit) mit C₂-C₂₄-Fettsäuren, z. B. das Isosorbidmonolaurat.
Beispiele für erfindungsgemäß besonders geeignete C₈-C₂₂-Fettsäureester mehrwertiger C₂-C₆-Alkanole (Typ b) sind als pflanzliche und tierische Öle vorkommende Fettsäuretriglycerid-Öle, z. B. Sonnenblumenöl, Sojaöl, Sesamöl, Haselnußöl, Mandelöl sowie Distelöl (Safloröl). Besonders bevorzugt ist Distelöl. Weniger geeignet ist z. B. Nachtkerzenöl (evening primrose oil), dessen Polarität so hoch ist, dass es selbst eine beträchtliche Grenzflächenaktivität zeigt. Nicht geeignet ist Olivenöl. Erfindungsgemäß außerordentlich gut geeignet sind weiterhin synthetische Fettsäuretriglycerid-Öle. Besonders bevorzugt sind die Triglyceride von gesättigten C₈-C₁₀-Fettsäuren, wobei die Fettsäuren sowohl unverzweigt, wie z. B. bei den Handelsprodukten Myritol^{®} 318 und Myritol^{®} 331 (Cognis) oder Miglyol^{®} 812 (Hüls), als auch verzweigt sein können, wie z. B. bei den Handelsprodukten Estol^{®} GTEH 3609 (Uniqema) oder Myritol^{®} GTEH (Cognis).
Weitere geeignete Ester dieses Typs sind die Fettsäureester des 1,2-Propylenglycols, des Neopentylglycols, z. B. Neopentylglycoldiheptanoat, des Trimethylolpropans und des Pentaerythrits, wobei die 1,2-Propylenglycolester bevorzugt sind. Beispiele für erfindungsgemäß bevorzugte Ölkomponenten sind die 1,2-Propylenglycoldiester von gesättigten, unverzweigten C₈-C₁₀-Fettsäuren, besonders bevorzugt die Handelsprodukte Myritol^{®} PC und Miglyol^{®} 840.
Beispiele für erfindungsgemäß besonders geeignete Fettsäureester von Polyglycerinen (Typ c) sind eolyglyceryl-2-dipolyhydroxystearat (Handelsprodukt Dehymuls^{®} PGPH von Cognis), Polyglyceryl-3-diisostearat (Handelsprodukte Lameform^{®} TGI von Cognis) und der Diester der Dilinolsäure mit Polyglyceryl-3-dilsostearat (Handelsprodukt Isolan^{®} PDI von Th. Goldschmidt).
Beispiele für erfindungsgemäß besonders geeignete Dicarbonsäureester (Typ d) sind Diisopropylsebacat, Diisopropyladipat, Dioctylmalat, Dioctylmaleat, Dicaprylylmaleat, Dioctyladipat, Di-n-butyladipat, Diisooctylsuccinat, Di-(2-hexyldecyl)-succinat und Diisotridecylacelaat.
Beispiele für erfindungsgemäß besonders geeignete Benzoesäureester von linearen oder verzweigten C₁₀-C₂₀-Alkanolen sind unter dem Warenzeichen Finsolv^{®} (Hersteller: Finetex) erhältlich. Besonders bevorzugt sind die Handelsprodukte Finsolv^{®} TN (Benzoesäure-C12-C15-alkylester), Finsolv^{®} SB (Benzoesäureisostearylester) und Finsolv^{®} BOD (Benzoesäureoctyldocecylester).

Als C₈-C₂₂-Fettalkoholester einwertiger oder mehrwertiger C₂-C₇-Hydroxycarbonsäuren sind die Ester der Glycolsäure, Milchsäure, Äpfelsäure, Weinsäure, Citronensäure und Salicylsäure erfindungsgemäß besonders geeignet. Solche Ester auf Basis von linearen C_{14/15}-Alkanolen und von in 2-Position verzweigten C_{12/13}-A/kanolen sind unter dem Warenzeichen Cosmacol^{®} von der Fa. Nordmann, Rassmann GmbH & Co, Hamburg, zu beziehen. Besonders bevorzugt sind die Handelsprodukte Cosrüäcol^{®} ESI, Cosmacol^{®} EMI und Cosmacol^{®} ETI. Diese Ester eignen sich nicht nur besonders gut zur Herstellung der erfindungsgemäßen Lipoprotein-Emulsionen, sondern weisen darüber hinaus auch weitere sehr erwünschte hautkosmetische Wirkungen auf, z. B. beschleunigen sie die Zellerneuerung und verlangsamen die Hautalterung.
Bevorzugte polare Ölkomponenten weisen bei 25° C eine Grenzflächenspannung (γ¹) gegen Wasser unter 30 mN/m auf. Besonders gut eignen sich als Ölkomponenten solche Öle oder Mischungen von Ölen, die eine Grenzflächenspannung (γ¹) zu Wasser bei 25° C im Bereich von 5 bis 20 mN/m, bevorzugt von 6 bis 15 mN/m aufweisen, also Öle mit einer mittleren bis höheren Polarität.
Die polaren Ölkomponenten sind in einer Menge von 1 - 20 Gew.-% in der erfindungsgemäßen Zusammensetzung enthalten. Bevorzugt ist ein Gehalt von 3 - 15 Gew.-%, besonders bevorzugt von 4 - 10 Gew.-% an polaren Ölkomponenten, bezogen auf die Gesamtzusammensetzung.

Die erfindungsgemäß verwendeten organischen UV-Filter sind ausgewählt aus den Derivaten von Dibenzoylmethan, Zimtsäureestem, Diphenylacrylsäureestern, Benzophenon, Campher, p-Aminobenzoesäureestem, o-Aminobenzoesäureestem, Salicylsäureestern, Benzimidazolen, 1,3,5-Triazinen, monomeren und oligomeren 4,4-Diarylbutadiencarbonsäureestern und -carbonsäureamiden, Ketotricyclo(5.2.1.0)decan, Benzalmalonsäureestern sowie beliebigen Mischungen der genannten Komponenten.
Die organischen UV-Filter können öllöslich oder wasserlöslich sein.
Erfindungsgemäß besonders bevorzugte öllösliche UV-Filter sind 1-(4-tert.-Butylphenyl)-3-(4'methoxyphenyl)propan-1,3-dion (z. B. Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone (Uvasorb^{®} HEB) sowie beliebige Mischungen der genannten Komponenten.

Bevorzugte wasserlösliche UV-Filter sind 2-Phenylbenzimidazol-5-sulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bomylidenmethyl) benzolsulfonsäure und 2-Methyl-5-(2-oxo-3-bornyliden)sulfonsäure und deren Salze.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtem herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsaticylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.
Die erfindungsgemäß eingesetzten anorganischen Lichtschutzpigmente sind ausgewählt aus feindispersen Metalloxiden und Metallsalzen, insbesondere Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die bevorzugten anorganischen Partikelsubstanzen sind hydrophil oder amphiphil. Vorteilhafterweise können sie oberflächlich beschichtet, d.h. hydrophilisiert oder hydrophobiert vorliegen. Bevorzugt sind die Pigmente oberflächlich wasserabweisend behandelt ("gecoatet"). Typische Beispiele sind gecoatete Titandioxide, wie z. B. Titandioxid T 805 (Degussa), oder mit Aluminiumstearat beschichtetes Titandioxid (Handelsprodukt MT 100 T von der Firma Tayca). Als hydrophobe Coatingmittel bevorzugt sind Silicone und dabei speziell Trialkoxyoctylsilane oder Simethicone. Bevorzugte Pigmente dieses Typs sind z. B. mit Dimethylpolysiloxan (Dimethicone) beschichtetes Zinkoxid, mit Dimethicone beschichtetes Bornitrid (Tres BN^{®} UHP 1106 von Carborundum), mit einem Gemisch aus Dimethylpolysiloxan und Silicagel (Simethicone) und Aluminiumoxidhydrat (Alumina) beschichtetes Titandioxid (Eusolex^{®} T 2000 von Merck), mit Octylsilanol beschichtetes Titandioxid oder sphärische Polyalkylsesquioxan-Partikel (Aerosil^{®} R972 und Aerosil^{®} 200V von Degussa).
Besonders bevorzugte anorganische Lichtschutzpigmente sind ausgewählt aus den Oxiden von Titan, Zink, Cer und Eisen sowie beliebigen Mischungen der genannten Komponenten, die oberflächenmodifiziert sein können.

In einer bevorzugten Ausführungsform enthalten die erfindungsgemäßen Zusammensetzungen mindestens ein Polymer. Die Polymere sind ausgewählt aus natürlichen und synthetischen anionischen und amphoteren Polymeren sowie aus synthetischen nichtionischen Polymeren. Erfindungsgemäß bevorzugt sind synthetische anionische, amphotere und nichtionische Polymere.
Erfindungsgemäß bevorzugte anionische Polymere enthalten Carboxylat- und/oder Sulfonatgruppen und als Monomere beispielsweise Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure. Ganz besonders bevorzugte anionische Polymere enthalten als einziges Monomer oder als Comonomer 2-Acrylamido-2-methylpropansulfonsäure, wobei die Sulfonsäuregruppe ganz oder teilweise in Salzform vorliegen kann. Besonders bevorzugt ist das Homopolymer der 2-Acrylamido-2-methylpropansulfonsäure, z. B. das Handelsprodukt Rheothik^{®}11-80.
Innerhalb dieser Ausführungsform kann es bevorzugt sein, Copolymere aus mindestens einem anionischen Monomer und mindestens einem nichtionischen Monomer einzusetzen. Bezüglich der anionischen Monomere wird auf die oben aufgeführten Substanzen verwiesen. Bevorzugte nichtionische Monomere sind Acrylamid, Methacrylamid, Acrylsäureester, Methacrylsäureester, Vinylpyrrolidon, Vinylether und Vinylester. Bevorzugte anionische Copolymere sind Acrylsäure-Acrylamid-Copolymere sowie insbesondere Polyacrylamidcopolymere mit Sulfonsäuregruppen-haltigen Monomeren. Ein besonders bevorzugtes anionisches Copolymer besteht aus 70 - 55 Mol-% Acrylamid und 30 - 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds hat sich im Rahmen der erfindungsgemäßen Lehre als besonders vorteilhaft erwiesen. Auch die unter der Bezeichnung Simulgel^{®} EG als Compound mit Isohexadecan und Polysorbat-80 vertriebenen Natriumacryloyldimethyltaurat-Copolymere haben sich als erfindungsgemäß besonders wirksam erwiesen.
Ebenfalls bevorzugte anionische Homopolymere sind unvernetzte und vernetzte Polyacrylsäuren. Dabei können Allylether von Pentaerythrit, von Sucrose und von Propylen bevorzugte Vemetzungsagentien sein. Solche Verbindungen sind beispielsweise die Handelsprodukte Carbopol^{®}. Weitere bevorzugte anionische Copolymere sind solche, die als Monomere 80 - 98 Gew.-% gewünschtenfalls substituierte Acrylsäure und 2 - 20 Gew.% C₁₂-C₃₀-Fettalkoholmethacrylsäureester enthalten und vernetzt sein können. Solche Verbindungen sind z. B. die Handelsprodukte Pemulen^{®}.
Unter amphoteren Polymeren versteht man sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO⁻- oder -SO⁻-Gruppen bzw. -COOH-oder SO₃H-Gruppen enthalten. Ein Beispiel für ein erfindungsgemäß einsetzbares amphoteres Polymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.
Die erfindungsgemäßen Mittel können weiterhin synthetische nichtionische Polymere enthalten. Geeignete nichtionische synthetische Polymere sind Polyvinylpyrrolidone und Vinylpyrrolidon/Vinylester-Copolymere, z. B. die Handelsprodukte Luviskol^{®} (BASF), sowie Polyvinylalkohole.

Erfindungsgemäß ebenfalls einsetzbar, wenn auch weniger bevorzugt, sind kationische Polymere. Man unterscheidet "temporär" und "permanent" kationische Polymere. Temporär kationische Polymere enthalten üblicherweise eine Aminogruppe, die bei bestimmten pH-Werten als quartäre Ammoniumgruppe vorliegt. Bevorzugt sind Chitosan und dessen Derivate, z. B. die Produkte Hydagen^{®} CMF, Hydagen^{®} HCMF, Kytame^{®} PC und Chitolam^{®} NB/101. Besonders gut geeignete Chitosane weisen einen Deacetylierungsgrad von wenigstens 80 % und ein Molekulargewicht von 5 10⁵ bis 5 10⁶ g/mol auf. Zur Herstellung erfindungsgemäßer Zubereitungen muß das Chitosan in die Salzform überführt werden. Hierzu geeignete Säuren sind z. B. Essigsäure, Glycolsäure, Weinsäure, Apfelsäure, Citronensäure, Milchsäure, 2-Pyrrolidon-5-carbonsäure, Benzoesäure und Salicylsäure.
Weitere verwendbare kationische Polymere sind beispielsweise quaternisierte CelluloseDerivate, z. B. die Handelsprodukte Celquat^{®} und Polymer JR^{®}, insbesondere Celquat^{®} H 100, Celquat^{®} L 200 und Polymer JR^{®}400, kationische Alkylpolyglycoside gemäß der DE-PS 44 13 686, kationisierter Honig, kationische Guar-Derivate, insbesondere die Produkte Cosmedia^{®}Guar und Jaguar^{®}, Polysiloxane mit quaternären Gruppen, wie z.B. die Handelsprodukte Q2-7224 (Dow Corning), Dow Coming^{®} 929 Emulsion, SM-2059 (General Electric), SLM-55067 (Wacker) sowie Abil^{®}-Quat 3270 und 3272 (Th. Goldschmidt), sowie die unter den Bezeichnungen Polyquaternium-2, Polyquaternium-17, Polyquaternium-18 und Polyquaternium-27 bekannten Polymeren mit quartären Stickstoffatomen in der Polymerhauptkette. Gleichfalls als kationische Polymere eingesetzt werden können die unter den Bezeichnungen Polyquaternium-24 bekannten Polymere.
Ebenfalls als kationisches Polymer verwendbar ist das, gewünschtenfalls vernetzte, Poly(methacryloyloxyethyltrimethylammoniumchlorid) mit der INCI-Bezeichnung Polyquaternium-37. Die Vernetzung kann mit Hilfe mehrfach olefinisch ungesättigter Verbindungen, beispielsweise Divinylbenzol, Tetraallyloxyethan, Methylenbisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens. Polyquatemlum-37 wird häufig in Form einer nichtwässrigen Polymerdispersion eingesetzt. Solche Polymerdispersionen sind unter den Bezeichnungen Salcare^{®} SC 95 und Salcare^{®} SC 96 im Handel erhältlich.
Copolymere aus Methacryloyloxyethyltrimethylammoniumchlorid und nichtionischen Monomeren, bevorzugt Acrylamid, Methacrylamid, Acrylsäure-C₁₋₄-alkylester und Methacrylsäure-C₁₋₄-alkylester, die gegebenenfalls vernetzt sein können, sind im Handel unter dem Namen Salcare^{®} SC 92 erhältlich.
Es ist erfindungsgemäß auch möglich, dass die verwendeten Zubereitungen mehrere, insbesondere zwei verschiedene Polymere gleicher Ladung und/oder jeweils ein ionisches und ein amphoteres und/oder ein synthetisches nichtionisches Polymer enthalten.

In einer weiteren bevorzugten Ausführungsform enthalten die erfindungsgemäßen Zusammensetzungen nichtionische Coemulgatoren vom Typ der polaren Fettstoffe oder der Wasser-in-Öl-Emulgatoren mit einem HLB-Wert unter 6,5. Bevorzugte Coemulgatoren sind lineare C₁₂-C₂₂-Fettatkohote und C₁₂-C₂₂-Fettsäurepartialester von C₂-C₆-Polyolen, deren HLB-Wert unter 6,5 liegt.
Geeignete Fettalkohole sind z.B. Cetylalkohol, Stearylalkohol, Oleylalkohol, Erucaalkohol, Ricinolalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole. Besonders bevorzugte Fettalkohole sind Cetylalkohol, Stearylalkohol oder ein Gemisch dieser beiden Fettalkohole, desweiteren Behenylalkohol sowie technische Gemische aus linearen C₂₀-C₂₂-Fettalkoholen.
Bevorzugte Polyol-Fettsäurepartialester sind Mono- und -diester von Glycerin oder 1,2-Propylenglycol mit gesättigten und ungesättigten linearen und verzweigten C₁₂-C₂₂-Fettsäuren sowie die Mono-, Sesqui- und -triester von Sorbitan mit gesättigten und ungesättigten linearen und verzweigten C₁₈-C₂₂-Fettsäuren. Besonders bevorzugt sind Glycerinmonostearat, Glycerinmonopalmitat, Glycerinmonolaurat, Glycerinmonooleat, Glycerindistearat, Glycerindipalmitat, Glycerindilaurat, Glycerindioleat sowie technische Gemische dieser Komponenten, weiterhin Sorbitanmonostearat, Sorbitanmonooleat, Sorbitansesquioleat, Sorbitantristearat, Sorbitantrioleat sowie technische Gemische dieser Komponenten. In den erfindungsgemäßen Zusammensetzungen beträgt das Gewichtsverhältnis von Coemulgator zu polarer Ölkomponente 0,1 : 1 bis 1 : 1.

In einer weiteren bevorzugten Ausführungsform enthalten die erfindungsgemäßen Zusammensetzungen mindestens ein Polyol. Bevorzugte Polyole sind Diole, Triole und höhere Alkohole, Polyglycerine, Polyethylenglycole sowie Mono- und Disaccharide. Unter den Diolen eignen sich C₂-C₁₂-Diole, insbesondere 1,2-Propylenglycol, Butylenglycole wie z. B. 1,2-Butylenglycol, 1,3-Butylenglycol und 1,4-Butylenglycol, Hexandiole, wie z. B. 1,6-Hexandiol. Weiterhin bevorzugt geeignet sind Glycerin und Polyglycerine, insbesondere Diglycerin und Triglycerin, 1,2,6-Hexantriol, sowie die Polyethylenglycole (PEG) PEG-400, PEG-600, PEG-1000, PEG-1550, PEG-3000 und PEG-4000.
Erfindungsgemäß geeignete Monosaccharide sind z. B. Glucose, Fructose, Galactose, Arabinose, Ribose, Xylose, Lyxose, Allose, Altrose, Mannose, Gulose, Idose und Talose, die Desoxyzucker Fucose und Rhamnose sowie Aminozucker wie z. B. Glucosamin oder Galactosamin. Bevorzugt sind Glucose, Fructose, Galactose, Arabinose und Fucose; Glucose ist besonders bevorzugt.
Erfindungsgemäß geeignete Disaccharide sind aus zwei Monosaccharideinheiten zusammengesetzt, z. B. Saccharose oder Lactose. Ein besonders bevorzugtes Disaccharid ist Saccharose. Ebenfalls besonders bevorzugt ist die Verwendung von Honig, der überwiegend Glucose und Saccharose enthält.

In einer weiteren bevorzugten Ausführungsform enthalten die erfindungsgemäßen Zusammensetzungen mindestens ein Vitamin, Pro-Vitamin, eine Vitaminvorstufe oder deren Derivate. Hierbei sind solche Komponenten bevorzugt, die üblicherweise den Vitamingruppen A, B, C, E, F und H zugeordnet werden.
Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen erfindungsgemäß beispielsweise Vitamin A-Säure und deren Ester, Vitamin A-Aldehyd und Vitamin A-Alkohol sowie dessen Ester, wie Retinylpalmitat und Retinylacetat in Betracht. Die erfindungsgemäß verwendeten Zubereitungen.enthalten die Vitamin A-Komponente bevorzugt in Mengen von 0,05 - 1 Gew.-%, bezogen auf die gesamte Zusammensetzung.

Zur Vitamin B-Gruppe oder zu dem Vitamin B-Komplex gehören u. a.
- Vitamin B₁ (Thiamin)
- Vitamin B₂ (Riboflavin)
- Vitamin B₃. Unter dieser Bezeichnung werden häufig die Verbindungen Nicotinsäure und Nicotinsäureamid (Niacinamid) geführt. Erfindungsgemäß bevorzugt ist das Nicotinsäureamid, das bevorzugt in Mengen von 0,05 bis 1 Gew.-%, bezogen auf die gesamte Zusammensetzung, enthalten ist.
- Vitamin B₅ (Pantothensäure und Panthenol). Bevorzugt wird Panthenol eingesetzt. Erfindungsgemäß einsetzbare Derivate des Panthenols sind insbesondere die Ester und Ether des Panthenols sowie kationisch derivatisierte Panthenole. In einer weiteren bevorzugten Ausführungsform der Erfindung können an Stelle von sowie zusätzlich zu Pantothensäure oder Panthenol auch Derivate des 2-Furanon mit der allgemeinen Strukturformel (I) eingesetzt werden. 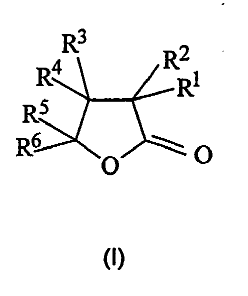 Bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄ - Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen. Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γbutyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (I) R¹ für eine Hydroxylgruppe, R² für ein Wasserstoffatom, R³ und R⁴ für eine Methylgruppe und R⁶ und R³ für ein Wasserstoffatom stehen. Das Stereoisomer (R)-Pantolacton entsteht beim Abbau von Pantothensäure.
   Die genannten Verbindungen des Vitamin B₅-Typs sowie die 2-Furanonderivate sind bevorzugt in einer Gesamtmenge von 0,05 bis 10 Gew.-%, besonders bevorzugt 0,1 bis 5 Gew.-%, jeweils bezogen auf die gesamte Zusammensetzung, enthalten.
- Vitamin B₆ (Pyridoxin sowie Pyridoxamin und Pyridoxal).
- Vitamin B₇ (Biotin), auch als Vitamin H oder "Hautvitamin" bezeichnet. Biotin ist bevorzugt in Mengen von 0,0001 bis 1,0 Gew.-%, insbesondere 0,001 bis 0,01 Gew.-%, jeweils bezogen auf die gesamte Zusammensetzung, enthalten.

Vitamin C (Ascorbinsäure) wird bevorzugt in Mengen von 0,1 bis 3 Gew.-%, bezogen auf die gesamte Zusammensetzung, eingesetzt. Die Verwendung der DerivateAscorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein. Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und Tocophersolan.Tocopherol und seine Derivate sind bevorzugt in Mengen von 0,05 - 1 Gew.-%, bezogen auf die gesamte Zusammensetzung, enthalten.
Unter dem Begriff "Vitamin F" werden üblicherweise essentielle Fettsäuren, insbesondere Linolsäure, Linolensäure und Arachidonsäure, verstanden.

Bevorzugt enthalten die erfindungsgemäßen Mittel Vitamine, Provitamine und Vitaminvorstufen aus den Gruppen A, B, C, E und H. Vitamin A-palmitat (Retinylpalmitat), Panthenol und seine Derivate, Nicotinsäureamid, Ascorbylpalmitat, -acetat, Mg-Ascorbylphosphat, Na-Ascorbylphosphat, Natrium- und Magnesiumascorbat, die Tocopherolester, besonders Tocopherylacetat, und Biotin sind besonders bevorzugt.

In einer weiteren bevorzugten Ausführungsform enthalten die erfindungsgemäßen Zusammensetzungen mindestens einen Pflanzenextrakt. Die erfindungsgemäß geeigneten Pflanzenextrakte werden üblicherweise durch Extraktion der gesamten Pflanze, in einzelnen Fällen aber auch ausschließlich aus Blüten und/oder Blättern und/oder Samen und/oder anderen Pflanzenteilen, hergestellt. Erfindungsgemäß bevorzugt sind vor allem die Extrakte aus dem Meristem, also dem teilungsfähigen Bildungsgewebe der Pflanzen, und speziellen Pflanzen wie Grünem Tee, Hamamelis, Kamille, Ringelblume, Stiefmütterchen, Paeonie, Aloe Vera, Rosskastanie, Salbei, Weidenrinde, Zimtbaum (cinnamon tree), Chrysanthemen, Eichenrinde, Brennessel, Hopfen, Klettenwurzel, Schachtelhalm, Weißdorn, Lindenblüten, Mandeln, Fichtennadeln, Sandelholz, Wacholder, Kokosnuß, Kiwi, Guave, Limette, Mango, Aprikose, Weizen, Melone, Orange, Grapefruit, Avocado, Rosmarin, Birke, Buchensprossen, Malve, Wiesenschaumkraut, Schafgarbe, Quendel, Thymian, Melisse, Hauhechel, Eibisch (Althaea), Malve (Malva sylvestris), Veilchen, Blättern der Schwarzen Johannisbeere, Huflattich, Fünffingerkraut, Ginseng, Ingwerwurzel und Süßkartoffel.
Vorteilhaft eingesetzt werden können auch Algenextrakte. Die erfindungsgemäß verwendeten Algenextrakte stammen aus Grünalgen, Braunalgen, Rotalgen oder Blaualgen (Cyanobakterien). Die zur Extraktion eingesetzten Algen können sowohl natürlichen Ursprungs als auch durch biotechnologische Prozesse gewonnen und gewünschtenfalls gegenüber der natürlichen Form verändert sein. Die Veränderung der Organismen kann gentechnisch, durch Züchtung oder durch die Kultivation in mit ausgewählten Nährstoffen angereicherten Medien erfolgen. Bevorzugte Algenextrakte stammen aus Seetang, Blaualgen, aus der Grünalge Codium tomentosum sowie aus der Braunalge Fucus vesiculosus. Ein besonders bevorzugter Algenextrakt stammt aus Blaualgen der Species Spirulina, die in einem Magnesium-angereicherten Medium kultiviert wurden.
Vorteilhaft eingesetzt werden können auch Extrakte aus Mikroorganismen, z. B. aus Hefen, bevorzugt aus Bäckerhefe.
Besonders bevorzugt sind die Extrakte aus Spirulina, Bäckerhefe, Grünem Tee, Aloe Vera, Meristem, Hamamelis, Aprikose, Ringelblume, Guave, Süßkartoffel, Limette, Mango, Kiwi, Gurke, Malve, Eibisch und Veilchen. Die erfindungsgemäßen Mittel können auch Mischungen aus mehreren, insbesondere aus zwei, verschiedenen Pflanzenextrakten enthalten.
Sebumregulierende Wirkstoffe werden im Sinne der Erfindung besonders bevorzugtausgewählt aus wasser- und öllöslichen Extrakten aus Hamamelis, Klettenwurzel und Brennessel, Zimtbaumextract (z. B. Sepicontrol^{®} A5 von der Firma Seppic), Chrysanthemenextrakt (z. B. Laricyl^{®} von Laboratoires Sérobiologiques) und aus handelsüblichen Wirkstoffmischungen, z. B. Asebiol^{®} BT 2 (INCI: Aqua, Hydrolyzed Yeast Protein, Pyridoxine, Niacinamide, Glycerin, Panthenol, Allantoin, Biotin) der Firma Laboratoires Sérobiologiques und Antifettfaktor^{®} COS-218/2-A (von Cosmetochem, INCI: Aqua, Cetyl-PCA, PEG-8 Isolauryl Thioether, PCA, Cetyl Alcohol).
Adstringierende Wirkstoffe werden im Sinne der Erfindung besonders bevorzugt ausgewählt aus wasser- und öllöslichen Extrakten aus Hamamelis, Weidenrinde, Eichenrinde, Fünffingerkraut, Spitzwegerich und Salbei.

Weiterhin können die erfindungsgemäßen Zusammensetzungen Antioxidantien enthalten, die sowohl zur Stabilisierung der Fette gegen Autoxidation als auch als Radikalfänger zur vorbeugenden Hautpflege dienen. Besonders vorteilhaft werden die Antioxidantien ausgewählt aus Aminosäuren (z. B. Glycin, Histidin, Tyrosin, Tryptophan) und deren Derivaten, Imidazol und Imidazolderivaten (z. B. Urocaninsäure), Peptiden wie z. B. D,L-Carnosin, D-Carnosin, L-Camosin und deren Derivaten (z. B. Anserin), Carotinoiden, Carotinen (z. B. α-Carotin, β-Carotin, Lycopin) und deren Derivaten, Liponsäure und deren Derivaten (z. B. Dihydroliponsäure), Aurothioglucose, Propylthiouracil und weiteren Thioverbindungen (z. B. Thioglycerin, Thiosorbitol, Thioglycolsäure, Thioredoxin, Glutathion, Cystein, Cystin, Cystamin und deren Glycosyl-, N-Acetyl-, Methyl-, Ethyl-, Propyl-, Amyl-, Butyl-, Lauryl-, Palmitoyl-, Oleyl-, γ-Linoleyl-, Cholesteryl- und Glycerylestern) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salzen) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol/kg bis µmol/kg), ferner Metallchelatoren (z. B. α-Hydroxyfettsäuren, EDTA, EGTA, Phytinsäure, Lactoferrin), α-Hydroxysäuren (z. B. Zitronensäure, Milchsäure, Äpfelsäure), Huminsäuren, Gallensäure, Gallenextrakten, Gallussäureestern (z. B. Propyl-, Octyl- und Dodecylgallat), Flavonoiden, Catechinen, Bilirubin, Biliverdin und deren Derivaten, ungesättigten Fettsäuren und deren Derivaten (z. B. γ-Linolensäure, Linolsäure, Arachidonsäure, Ölsäure), Folsäure und deren Derivaten, Hydrochinon und dessen Derivaten (z. B. Arbutin), Ubichinon und Ubichinol sowie deren Derivaten, Vitamin C und dessen Derivaten, Isoascorbinsäure und deren Derivaten, Tocopherolen und deren Derivaten, Vitamin A und Derivaten, dem Coniferylbenzoat des Benzoeharzes, Rutin, Rutinsäure und deren Derivaten (z. B. Dinatriumrutinyldisulfat), Zimtsäure und deren Derivaten (z. B. Ferulasäure, Ethylferulat, Kaffeesäure), Kojisäure, Chitosanglycolat und -salicylat, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und Zink-Derivaten (z. B. ZnO, ZnSO₄), Selen und Selen-Derivaten (z. B. Selenmethionin) sowie Stilbenen und Stilben-Derivaten (z. B. Stilbenoxid, trans-Stilbenoxid).
Erfindungsgemäß können geeignete Derivate (Salze, Ester, Zucker, Nukleotide, Nukleoside, Peptide und Lipide) sowie Mischungen dieser genannten Wirkstoffe oder Pflanzenextrakte (z. B. Teebaumöl, Rosmarinextrakt und Rosmarinsäure), die diese Antioxidantien enthalten, eingesetzt werden.
Die Gesamtmenge der Antioxidantien in den erfindungsgemäßen Zusammensetzungen beträgt 0,001 - 10 Gew.-%, vorzugsweise 0,05 - 5 Gew.-% und insbesondere 0,1 - 2 Gew.-%, bezogen auf das Gesamtgewicht der Zubereitung.

Die erfindungsgemäßen Zusammensetzungen können außer den vorgenannten polaren Ölkomponenten und den Coemulgatoren auch unpolare Ölkomponenten sowie Fettstoffe, die bei Raumtemperatur fest sind, enthalten. Ihr Anteil an der Gesamtmenge der Öl-, Fett- und Coemulgatorphase liegt bei 1 - 50 %, bevorzugt 5 - 30 %. Zu diesen übrigen Öl- und Fettkomponenten zählen Fettsäuren sowie natürliche und synthetische Wachse, die sowohl in fester Form als auch flüssig oder in wäßriger Dispersion vorliegen können, sowie natürliche und synthetische unpolare kosmetische Öle.
Als Fettsäuren können eingesetzt werden lineare und/oder verzweigte, gesättigte und/oder ungesättigte C₈₋₃₀-Fettsäuren. Bevorzugt sind C₁₀₋₂₂-Fettsäuren. Beispiele sind die Isostearinsäuren und Isopalmitinsäuren. Weitere geeignete Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachidonsäure, Gadoleinsäure, Behensäure und Erucasäure sowie deren technische Mischungen. Besonders bevorzugt sind üblicherweise die Fettsäureschnitte, welche aus Cocosöl oder Palmöl erhältlich sind; insbesondere bevorzugt ist der Einsatz von Stearinsäure.
Als natürliche oder synthetische Wachse können erfindungsgemäß eingesetzt werden feste Paraffine oder Isoparaffine, Pflanzenwachse wie Candelillawachs, Carnaubawachs, Espartograswachs, Fruchtwachse und Sonnenblumenwachs, Bienenwachse und andere Insektenwachse, Ozokerite, Ceresin, Walrat sowie Microwachse aus Polyethylen oder Polypropylen. Weiterhin geeignet sind die Triglyceride gesättigter und gegebenenfalls hydroxylierter C₁₆₋₃₀-Fettsäuren, wie z. B. gehärtete Triglyceridfette (hydriertes Palmöl, hydriertes Kokosöl, hydriertes Rizinusöl), Glyceryltribehenat oder Glyceryltri-12-hydroxystearat, weiterhin synthetische Vollester aus Fettsäuren und Glykolen (z. B. Syncrowachs^{®}) oder Polyolen mit 2 - 6 C-Atomen, Fettsäuremonoalkanolamide mit einem C₁₂₋₂₂ -Acylrest und einem C₂₋₄-Alkanolrest, synthetische Fettsäure-Fettalkoholester, z. B. Stearylstearat oder Cetylpalmitat, Esterwachse aus natürlichen Fettsäuren und synthetischen C₂₀₋₄₀-Fettalkoholen (INCI-Bezeichnung C20-40 Alkyl Stearate) und Vollester aus Fettalkoholen und Di- und Tricarbonsäuren, z. B. Dicetylsuccinat oder Dicetyl-/stearyladipat, sowie Mischungen dieser Substanzen.
Vorteilhafte kosmetische Ölkörper, die erfindungsgemäß eingesetzt werden können, sind beispielsweise flüssige Paraffinöle, Isoparaffinöle und synthetische Kohlenwasserstoffe wie 1,3-Di-(2-ethyl-hexyl)-cyclohexan (Cetiol^{®} S) sowie Di-n-alkylether mit insgesamt 12 bis 36, insbesondere 12 bis 24 C-Atomen, wie Di-n-octylether, Di-n-decylether, Di-n-nonylether, Di-n-undecylether, n-Hexyl-n-octylether und n-Octyl-n-decylether. 1,3-Di-(2-ethyl-hexyl)-cyclohexan (Cetiol^{®} S) und Di-n-octylether (Cetiol^{®} OE) können bevorzugt sein. Weiterhin können symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC) eingesetzt werden.
Weitere erfindungsgemäß vorteilhaft einzusetzende Fettstoffe sind Siloxane. Die Siloxane können als Öle, Harze oder Gums vorliegen. Bevorzugte Siloxane sind Polydialkylsiloxane, wie z. B. Polydimethylsiloxan, Polyalkylarylsiloxane, wie z. B. Polyphenylmethylsiloxan, Polydialkylsiloxane, die Amin- und/oder Hydroxy-Gruppen enthalten sowie cyclische Silicone (INCI-Bezeichnung: Cyclomethicone), bevorzugt Octamethylcyclotetrasiloxan, Decamethylcyclopentasiloxan und Dodecamethylcyclohexasiloxan.

Besonders bevorzugte Komponenten mit kosmetischer und dermatologischer Wirkung sind außerdem Allantoin, Bisabolol, Pyrrolidoncarbonsäure sowie Sterine, z. B. Ergosterin, Stigmasterin, Sitosterin und Mykosterine.
Als weitere Hilfsmittel können die erfindungsgemäßen Zusammensetzungen übliche galenische Hilfsstoffe in den gebräuchlichen Mengen enthalten. Solche Hilfsstoffe sind z.B. Verdickungsmittel für die wässrige Phase, z.B. vom Typ der Schichtsilikate oder der pyrogenen Kieselsäuren. Weitere Hilfsstoffe sind Konservierungsstoffe wie z. B. Phenoxyethanol, p-Hydroxybezoesäureester, Glycin oder Sorbinsäure, Komplexbildner (z.B. Trilon B) sowie Farb- und Duftstoffe.
Die folgenden Beispiele sollen den Gegenstand der Erfindung näher erläutern, ohne ihn hierauf zu beschränken.

### Beispiele

### 1. Untersuchung der Grenzflächenspannung zwischen Ölkomponenten und Wasser bzw. 1 Gew.-%igen Lösungen von Proteinen in Wasser

### Proteinpräparate:

| | | |
|---|---|---|
| P1 : | COS-152-11-A (Cosmetochem) : | Haferprotein |
| P2 : | COS-152-15-A (Cosmetochem) : | Weizenprotein |
| P3 : | COS-151-2-A (Cosmetochem) : | Erbsenprotein |

### Messung der Grenzflächenspannung:

| **Ölkomponente** | **Grenzflächenspannung (mN/m) 25° C** | |
|---|---|---|
| | **Wasser (**γ**¹)** | **1 Gew.-% Protein in Wasser (**γ**²)** |
| Kessco^{®} IPS | 27,8 | 15,5 (Protein P1) |
| | | 15,8 (Protein P2) |
| | | 11,4 (Protein P3) |

| **Ölkomponente** | **Grenzflächenspannung (mN/m) 25° C** | |
|---|---|---|
| | **Wasser (**γ**¹)** | **1 Gew.-% Protein in Wasser (**γ**²)** |
| Kessco^{®} IPS/Myritol^{®} PC im | 24,8 | Protein P1 : 15,3 |
| Gewichtsverhältnis 1:1 | | |
| | | Protein P2 : 13,4 |
| | | Protein P3 : 8,9 |

Die Messungen wurden mit einem Ringtensiometer durchgeführt.

### 2. Erfindungsgemäße Beispielrezepturen (Mengenangaben in Gewichtsteilen)

| | | **2.1** | **2.2** | **2.3** | **2.4** | **2.5** | **2.6** | **2.7** | **2.8** |
|---|---|---|---|---|---|---|---|---|---|
| Phase 1 | Distelöl | 3,0 | 3,0 | 3,0 | 3,0 | 3,0 | 3,0 | 3,0 | 3,0 |
| | Myritol^{®} PC | 3,5 | 3,5 | 3,5 | 3,5 | 3,5 | 3,5 | 3,5 | 3,5 |
| | Lanette^{®} 22 | 3,0 | 3,0 | 3,0 | 3,0 | 3,0 | 3,0 | 3,0 | 3,0 |
| | Cutina^{®} GMS-V | 3,0 | 3,0 | 3,0 | 3,0 | 3,0 | 3,0 | 3,0 | 3,0 |
| | Stenol^{®} 16/18 | 2,0 | 2,0 | 2,0 | 2,0 | 2,0 | 2,0 | 2,0 | 2,0 |
| | Isopropylstearat | 6,0 | 6,0 | 6,0 | 6,0 | 6,0 | 6,0 | 6,0 | 6,0 |
| | Baysiton^{®} M350 | 1,0 | 1,0 | 1,0 | 1,0 | 1,0 | 1,0 | 1,0 | 1,0 |
| | Controx^{®} KS | 0,05 | 0,05 | 0,05 | 0,05 | 0,05 | 0,05 | 0,05 | 0,05 |
| | Parsol^{®} 1789 | 2,0 | 2,0 | - | 1,0 | 1,0 | 2,0 | 2,0 | - |
| | Parsol^{®} MCX | 3,0 | 3,0 | - | - | 7,5 | 3,0 | 3,0 | - |
| | Eusolex^{®} 4360 | - | - | 0,5 | - | - | - | - | 0,5 |
| | Eusolex^{®} 6300 | 3,0 | 3,0 | 2,0 | 2,0 | - | 3,0 | 3,0 | 2,0 |
| | Uvinul^{®} T 150 | 2,5 | 2,5 | - | - | - | 2,5 | 2,5 | - |
| | Propylparaben | 0,2 | 0,2 | 0,2 | 0,2 | 0,2 | 0,2 | 0,2 | 0,2 |
| Phase 2 | Glycerin | 5,0 | 5,0 | 5,0 | 5,0 | 5,0 | 5,0 | 5,0 | 5,0 |
| | Methylparaben | 0,2 | 0,2 | 0,2 | 0,2 | 0,2 | 0,2 | 0,2 | 0,2 |
| | Wasser | 54,95 | 48,95 | 58,95 | 56,45 | 50,95 | 54,95 | 48,95 | 56,95 |
| Phase 3 | V-Protein flüssig (COS 152/22-A) | - | 9,0 | 9,0 | 9,0 | 9,0 | - | 9,0 | 9,0 |
| | Hibiscin^{®} HP-LS-9198 | 3,0 | - | - | - | - | 3,0 | - | - |
| Phase 4 | Mirasun^{®} TIW 60 | - | - | - | - | - | - | 2,5 | - |
| | Granlux^{®} MSN 50 | 2,5 | 2,5 | - | 2,5 | - | 2,5 | - | - |
| | Eusolex^{®} T-Aqua | - | - | - | - | 2,5 | - | - | 2,5 |
| Phase 5 | Citronensäure | 0,1 | 0,1 | 0,1 | 0,1 | 0,1 | 0,1 | 0,1 | 0,1 |
| Phase 6 | Sepigel^{®} 305 | 2,0 | 2,0 | 2,0 | - | - | 2,0 | 2,0 | 2,0 |
| | Simulgel^{®} EG | - | - | - | 2,0 | 2,0 | - | - | - |

### Herstellung:

Fett- und Wasserphase (Phase 1 und Phase 2) wurden getrennt voneinander auf etwa 80° C erhitzt. Die Wasserphase wurde unter dem Homogenisator vorgelegt. Dann wurden die Proteinlösung und das Titandioxid zugegeben. Anschließend wurde etwa 1 Minute lang homogenisiert. Dann wurde die Fettphase hinzugegeben und erneut etwa 1 Minute lang homogenisiert. Nach dem Abkühlen auf etwa 30° C wurde der pH-Wert mit Citronensäure auf einen Wert im Bereich von 4,8 - 5,2 eingestellt. Anschließend wurde das Polymer (Sepigel^{®} 305 oder Simulgel^{®} EG) hinzugegeben. Es bildete sich eine stabile Emulsion, nach dem Abkühlen auf 20° C eine glatte Creme von hoher Stabilität.

### Liste der verwendeten Inhaltsstoffe

| **Komponente** | **INCl-Bezeichung** | | **Hersteller** |
|---|---|---|---|
| Kessco^{®} IPS | Isopropyl Stearate | | Akzo Nobel |
| Myritol^{®} PC | Propylene Glycol Dicaprylate/Dicaprate | | Cognis |
| Lanette^{®} 22 | Behenyl Alcohol | | Cognis |
| Cutina^{®} GMS-V | Glyceryl Stearate | Glycerin-mono-di-palmitatstearat, pflanzlich | Cognis |
| Stenol^{®} 16/18 | Cetearyl Alcohol | Cetyl-/Stearylalkohol (1 : 2) | Cognis |
| Baysilonöl^{®} M350 | Dimethicone | Polydimethylsiloxan | GE-Bayer-Silicones |
| Controx^{®} KS | Tocopherol, Hydrogenated Palm Glycerides Citrate | Tocopherol-Monodiglyceride-citrat-Gemisch | Cognis |
| Parsol^{®} 1789 | Butyl Methoxydibenzoylmethane | | Roche-Givaudan |
| Parsol^{®} MCX | Octyl Methoxycinnamate | | Roche-Givaudan |
| Eusolex^{®} 4360 | Benzophenone-3 | | Merck |
| Eusolex^{®} 6300 | 4-Methylbenzylidene Camphor | | Merck |
| Uvinul^{®} T 150 | Octyl Triazone | | BASF |
| V-Protein flüssig (COS 152/22-A) | Water, Propylene Glycol, Hydrolyzed Pea Protein (Pisum sativum) | | Cosmetochem |
| Hibiscin^{®} HP-LS-9198 | Water, Hibiscus esculentus Seed Extract, Phenoxyethanol | | Laboratoires Sérobiologiques |
| Mirasun^{®} TIW 60 | Titanium Dioxide | Wässrige Dispersion mit 40 Gew.-% TiO₂, mit Kieselgel und Aluminiumoxiden oberflächenbehandelt, Partikelgröße 60 nm | Rhône-Poulenc |
| Granlux^{®} MSN 50 | Titanium dioxide, Apricot Kernel Oil PEG-40 Esters, Cetearyl Glucoside, Polydecene | Dispersion mit 45 - 55 Gew.-% TiO₂, Partikelgröße 22 nm | Oy Granula AB, Finnland |
| Eusolex^{®} T-Aqua | Water, Titanium Dioxide, Alumina, Sodium Metaphosphate, Phenoxy-ethanol, Sodium Methylparaben | | Merck |
| Sepigel^{®} 305 | Polyacrylamide, C13-14 Isoparaffin, Laureth-7 | | SEPPIC |
| Simulgel^{®} EG | Sodium Acrylate/ Sodium Acryloyldimethyl Taurate Copolymer, Isohexadecane, Polysorbate 80 | | SEPPIC |

## Patentansprüche

1. Kosmetische und pharmazeutische Zusammensetzungen mit UV-Schutz in Form einer Öl-in-Wasser-Emulsion, die
a) mindestens ein gelöstes pflanzliches Protein oder Proteinhydrolysat, dessen wässrige Lösung in einer Konzentration von 1 Gew.-% gegenüber der Ölkomponente eine Grenzflächenspannung bei 25° C (γ²) aufweist, die niedriger ist als die Grenzflächenspannung γ¹ zwischen Ölkomponente und Wasser,
b) mindestens eine polare Ölkomponente, ausgenommen Olivenöl,
c) mindestens einen organischen oder anorganischen UV-Filter sowie Mischungen hiervon
enthält und die
d) frei ist von ionischen Emulgatoren, alkoxylierten Emulgatoren und Emulgatoren mit einem HLB-Wert über 6,5.

2. Zusammensetzungen gemäß Anspruch 1, **dadurch gekennzeichnet, dass** das pflanzliche Protein oder Proteinhydrolysat gewonnen ist aus den Samen von Hibiscus esculentus, aus Erbsen, Hafer oder Weizen.

3. Zusammensetzungen gemäß Anspruch 1 oder 2, **dadurch gekennzeichnet, dass**, bezogen auf die Gesamtzusammensetzung, 30 bis 75 Gew.-% Wasser enthalten sind.

4. Zusammensetzungen gemäß Anspruch 1-3, **dadurch gekennzeichnet, dass** die polare Ölkomponente ausgewählt ist aus den Estern von gesättigten und ungesättigten, linearen und verzweigten C₃-C₂₂-Fettsäuren und den Dimeren ungesättigter C₁₂-C₂₂-Fettsäuren (Dimerfettsäuren) mit einwertigen linearen, verzweigten oder cyclischen C₂-C₁₈-Alkanolen, mehrwertigen linearen oder verzweigten C₂-C₆-Alkanolen, Polyglycerinen der Formel CH₂OH-CHOH-CH₂[-O-CH₂-CHOH-CH₂]ₙ-O-CH₂-CHOH-CH₂OH mit n = 0- 8, und den Dicarbonsäureestern von linearen oder verzweigten C₂-C₁₀-Alkanolen, den Benzoesäureestern von linearen oder verzweigten C₁₀-C₂₀-Alkanolen, den C₁₂-C₂₂-Alkanolestem ein- oder mehrwertiger C₂-C₇-Hydroxycarbonsäuren, die aromatisch sein können, sowie Mischungen dieser Ölkomponenten, ausgenommen Olivenöl.

5. Zusammensetzungen gemäß Anspruch 1-4, **dadurch gekennzeichnet, dass** die polare Ölkomponente ausgewählt ist aus pflanzlichen, tierischen und synthetischen Fettsäuretriglycerid-Ölen, ausgenommen Nachtkerzenöl und Olivenöl.

6. Zusammensetzungen gemäß einem der Ansprüche 1-5, **dadurch gekennzeichnet, dass** die polare Ölkomponente bei 25° C eine Grenzflächenspannung (γ¹) gegen Wasser unter 30 mN/m aufweist.

7. Zusammensetzungen gemäß Anspruch 1-6, **dadurch gekennzeichnet, dass** die organischen UV-Filter ausgewählt sind aus Derivaten von Dibenzoylmethan, Zimtsäureestern, Diphenylacrylsäureestem, Benzophenon, Campher, p-Aminobenzoesäureestern, o-Aminobenzoesäureestern, Salicylsäureestern, Benzimidazolen, 1,3,5-Triazinen, monomeren und oligomeren 4,4-Diarylbutadiencarbonsäureestem und -carbonsäureamiden, Ketotricyclo(5.2.1.0)decan, Benzalmalonsäureestern sowie beliebigen Mischungen der genannten Komponenten.

8. Zusammensetzungen gemäß Anspruch 1-7, **dadurch gekennzeichnet, dass** die organischen UV-Filter ausgewählt sind aus 1-(4-tert.-Butylphenyl)-3-(4'-methoxyphenyl)propan-1,3-dion, 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone) und Dioctyl Butamido Triazone, (Uvasorb^{®} HEB) sowie beliebige Mischungen der genannten Komponenten.

9. Zusammensetzungen gemäß Anspruch 1-8, **dadurch gekennzeichnet, dass** die anorganischen UV-Filter ausgewählt sind aus Oxiden von Titan, Zink, Cer und Eisen sowie beliebigen Mischungen der genannten Komponenten, die oberflächenmodifiziert sein können.

10. Zusammensetzungen gemäß einem der Ansprüche 1 bis 9, **dadurch gekennzeichnet, dass** weiterhin mindestens ein Polymer enthalten ist.

11. Zusammensetzungen gemäß Anspruch 10, **dadurch gekennzeichnet, dass** das Polymer ausgewählt ist aus natürlichen und synthetischen anionischen und amphoteren Polymeren sowie aus synthetischen nichtionischen Polymeren.

12. Zusammensetzungen gemäß einem der Ansprüche 1 bis 11, **dadurch gekennzeichnet, dass** weiterhin mindestens ein nichtionischer Coemulgator vom Typ der polaren Fettstoffe oder der Wasser in-Öl-Emulgatoren mit einem HLB-Wert unter 6,5 enthalten ist.

13. Zusammensetzungen gemäß Anspruch 12, **dadurch gekennzeichnet, dass** der Coemulgator mit einem HLB-Wert unter 6,5 ausgewählt ist aus linearen C₁₂-C₂₂-Fettalkoholen und C₁₂-C₂₂-Fettsäurepartialestem von C₂-C₆-Polyolen.

14. Zusammensetzungen gemäß Anspruch 12 oder 13, **dadurch gekennzeichnet, dass** das Gewichtsverhältnis von Coemulgator zu polarer Ölkomponente 0,1 : 1 bis 1 : 1 beträgt.

15. Zusammensetzungen gemäß einem der Ansprüche 1 bis 14, **dadurch gekennzeichnet, dass** weiterhin mindestens ein Vitamin, Pro-Vitamin, eine Vitaminvorstufe oder deren Derivate aus den Vitamingruppen A, B, C, E, F und H enthalten ist.

## Claims

1. Cosmetic and pharmaceutical compositions with UV-protection in the form of an oil-in-water emulsion, which contains
a) at least one dissolved plant protein or protein hydrolysate, an aqueous solution of which in a concentration of 1 wt.% exhibits at 25°C an interfacial tension relative to the oil component (γ²) which is lower than the interfacial tension γ¹ between the oil component and water,
b) at least one polar oil component, with the exception of olive oil,
c) at least one organic or inorganic UV filter and mixtures thereof and which
d) contains no ionic emulsifiers, alkoxylated emulsifiers and emulsifiers with an HLB value of above 6.5.

2. Compositions according to claim 1, **characterised in that** the plant protein or protein hydrolysate is obtained from the seeds of *Hibiscus esculentus*, from peas, oats or wheat.

3. Compositions according to claim 1 or claim 2, **characterised in that**, relative to the total composition, they contain 30 to 75 wt.% water.

4. Compositions according to claims 1-3, **characterised in that** the polar oil component is selected from the esters of saturated and unsaturated, linear and branched C₃-C₂₂ fatty acids and the dimers of unsaturated C₁₂-C₂₂ fatty acids (dimer fatty acids) with monovalent linear, branched or cyclic C₂-C₁₈ alkanols, polyhydric linear or branched C₂-C₆ alkanols, polyglycerols of the formula CH₂OH-CHOH-CH₂[-O-CH₂-CHOH-CH₂]ₙ-O-CH₂-CHOH-CH₂OH with n = 0-8, and the dicarboxylic acid esters of linear or branched C₂-C₁₀ alkanols, the benzoic acid esters of linear or branched C₁₀-C₂₀ alkanols, the C₁₂-C₂₂ alkanol esters of mono- or polyvalent C₂-C₇ hydroxycarboxylic acids, which may be aromatic, and mixtures of these oil components, with the exception of olive oil.

5. Compositions according to claims 1-4, **characterised in that** the polar oil component is selected from plant, animal and synthetic fatty acid triglyceride oils, with the exception of evening primrose oil and olive oil.

6. Compositions according to any one of claims 1-5, **characterised in that**, the polar oil component exhibits at 25°C an interfacial tension (γ¹) relative to water of below 30 mN/m.

7. Compositions according to claims 1-6, **characterised in that** the organic UV filters are selected from derivatives of dibenzoylmethane, cinnamic esters, diphenylacrylic esters, benzophenone, camphor, p-aminobenzoic esters, o-aminobenzoic esters, salicylic esters, benzimidazoles, 1,3,5-triazines, monomeric and oligomeric 4,4-diarylbutadiene carboxylic esters and -carboxamides, ketotricyclo(5.2.1.0)decane, benzalmalonic esters and any desired mixtures of the stated components.

8. Compositions according to claims 1-7, **characterised in that** the organic UV filters are selected from 1-(4-tert.-butylphenyl)-3-(4'-methoxyphenyl)-propane-1,3-dione, 1-phenyl-3-(4'-isopropylphenyl)-propane-1,3-dione, 3-(4'-methylbenzylidene)-D,L-camphor, 4-(dimethylamino)benzoic acid 2-ethylhexyl ester, 4-(dimethylamino)benzoic acid 2-octyl ester, 4-(dimethylamino)benzoic acid amyl ester, 4-methoxycinnamic acid 2-ethylhexyl ester, 4-methoxycimmanic acid propyl ester, 4-methoxycinnamic acid isopentyl ester, 2-cyano-3,3-phenylcinnamic acid 2-ethylhexyl ester (Octocrylene), salicylic acid 2-ethylhexyl ester, salicylic acid 4-isopropylbenzyl ester, salicylic acid homomenthyl ester (3,3,5-trimethylcyclohexyl salicylate), 2-hydroxy-4-methoxybenzophenone, 2-hydroxy-4-methoxy-4'-methylbenzophenone, 2,2'-dihydroxy-4-methoxybenzophenone, 4-methoxybenzylmalonic acid di-2-ethylhexyl ester, 2,4,6-trianilino-(p-carbo-2'-ethyl-1'-hexyloxy)-1,3,5-triazine (Octyl Triazone) and Dioctyl Butamido Triazone (Uvasorb® HEB) and any desired mixtures of the stated components.

9. Compositions according to claims 1-8, **characterised in that** the inorganic UV filters are selected from oxides of titanium, zinc, cerium and iron and any desired mixtures of the stated components, which may be surface-modified.

10. Compositions according to any one of claims 1 to 9, **characterised in that** they furthermore contain at least one polymer.

11. Compositions according to claim 10, **characterised in that** the polymer is selected from natural and synthetic anionic and amphoteric polymers and from synthetic nonionic polymers.

12. Compositions according to any one of claims 1 to 11, **characterised in that** they furthermore contain at least one nonionic coemulsifier of the polar fatty substance type or of the water-in-oil emulsifier type with an HLB value of below 6.5.

13. Compositions according to claim 12, **characterised in that** the coemulsifier with an HLB value of below 6.5 is selected from linear C₁₂-C₂₂ fatty alcohols and C₁₂-C₂₂ fatty acid partial esters of C₂-C₆ polyols.

14. Compositions according to claim 12 or claim 13, **characterised in that** the ratio by weight of coemulsifier to more highly polar oil component amounts to 0.1:1 to 1:1.

15. Compositions according to any one of claims 1 to 14, **characterised in that** they furthermore contain at least one vitamin, provitamin, a vitamin precursor or the derivatives thereof from the vitamin A, B, C, E, F and H groups.

## Revendications

1. Compositions cosmétiques et pharmaceutiques présentant une protection aux UV sous forme d'une émulsion huile-dans-eau, qui contient
a) au moins une protéine végétale dissoute ou un hydrolysat de protéines, dont la solution aqueuse à une concentration de 1% en poids présente par rapport au composant huileux, une tension interfaciale à 25° C (γ²), qui est inférieure à la tension interfaciale γ¹ entre le composant huileux et l'eau,
b) au moins un composant huileux polaire, à l'exception de l'huile d'olive,
c) au moins un filtre des UV organique ou inorganique ainsi que des mélanges de ceux-ci
et qui
d) est exempte d'émulsifiants ioniques, d'émulsifiants alcoxylés et d'émulsifiants présentant une valeur BHL supérieure à 6,5.

2. Compositions selon la revendication 1, **caractérisées en ce que** la protéine végétale ou l'hydrolysat de protéine est obtenu à partir des graines d'Hibiscus esculentus, de pois, d'avoine ou de blé.

3. Compositions selon la revendication 1 ou 2, **caractérisées en ce que**, par rapport à la composition totale, elles contiennent 30 à 75% en poids d'eau.

4. Compositions selon la revendication 1 à 3, **caractérisées en ce que** le composant huileux polaire est choisi parmi les esters d'acides gras en C₃-C₂₂ saturés et insaturés, linéaires et ramifiés et les dimères d'acides gras en C₁₂-C₂₂ insaturés (acides gras dimères) avec des alcanols en C₂-C₁₈ monovalents linéaires, ramifiés ou cycliques, des alcanols en C₂-C₆ polyvalents linéaires ou ramifiés, des polyglycérols de formule CH₂OH-CHOH-CH₂[-O-CH₂-CHOH-CH₂]ₙ-O-CH₂-CHOH-CH₂OH avec n = 0 - 8, et les esters d'acides dicarboxyliques d'alcanols en C₂-C₁₀ linéaires ou ramifiés, les esters de l'acide benzoïque d'alcanols en C₁₀-C₂₀ linéaires ou ramifiés, les esters d'alcanols en C₁₂-C₂₂ d'acides C₂-C₇-hydroxycarboxyliques monovalents ou polyvalents, qui peuvent être aromatiques, ainsi que les mélanges de ces composants huileux, à l'exception de l'huile d'olive.

5. Compositions selon la revendication 1 à 4, **caractérisées en ce que** le composant huileux polaire est choisi parmi les huiles de triglycérides d'acides gras végétaux, animaux et synthétiques, à l'exception de l'huile d'onagre et de l'huile d'olive.

6. Compositions selon l'une quelconque des revendications 1 à 5, **caractérisées en ce que** le composant huileux polaire à 25°C présente une tension interfaciale (γ¹) par rapport à l'eau inférieure à 30 mN/m.

7. Compositions selon la revendication 1 à 6, **caractérisées en ce que** les filtres des UV organiques sont choisis parmi les dérivés du dibenzoylméthane, des esters de l'acide cinnamique, des esters de l'acide diphénylacrylique, de la benzophénone, du camphre, des esters de l'acide p-aminobenzoïque, des esters de l'acide o-aminobenzoïque, des esters de l'acide salicylique, des benzimidazoles, des 1,3,5-triazines, des esters de l'acide 4,4-diarylbutadiènecarboxylique monomères et oligomères et des amides de l'acide 4,4-diarylbutadiènecarboxylique monomères et oligomères, du cétotricyclo(5.2.1.0)décane, des esters de l'acide benzalmalonique ainsi que des mélanges quelconques des composants mentionnés.

8. Compositions selon la revendication 1 à 7, **caractérisées en ce que** les filtres des UV organiques sont choisis parmi la 1-(4-tert-butylphényl)-3-(4'-méthoxyphényl)propane-1,3-dione, la 1-phényl-3-(4'-isopropylphényl)-propane-1,3-dione, le 3-(4'-méthylbenzylidène)-D,L-camphre, l'ester 2-éthylhexylique de l'acide 4-(diméthylamino)-benzoïque, l'ester 2-octylique de l'acide 4-(diméthylamino)benzoïque, l'ester amylique de l'acide 4-(diméthylamino)-benzoïque, l'ester 2-éthylhexylique de l'acide 4-méthoxycinnamique, l'ester propylique de l'acide 4-méthoxycinnamique, l'ester isopentylique de l'acide 4-méthoxycinnamique, l'ester 2-éthylhexylique de l'acide 2-cyano-3,3-phénylcinnamique (Octocrylene), l'ester 2-éthylhexylique de l'acide salicylique, l'ester 4-isopropylbenzylique de l'acide salicylique, l'ester homomenthylique de l'acide salicylique (salicylate de 3,3,5-triméthyl-cyclohexyle), la 2-hydroxy-4-méthoxybenzophénone, la 2-hydroxy-4-méthoxy-4'-méthylbenzophénone, la 2,2'-dihydroxy-4-méthoxybenzophénone, l'ester di-2-éthylhexylique de l'acide 4-méthoxybenzylmalonique, la 2,4,6-trianilino-(p-carbo-2'-éthyl-1'-hexyloxy)-1,3,5-triazine (Octyl Triazone) et le Dioctyl Butamido Triazone (Uvasorb® HEB) ainsi que des mélanges quelconques des composants mentionnés.

9. Compositions selon la revendication 1 à 8, **caractérisées en ce que** les filtres des UV inorganiques sont choisis parmi des oxydes du titane, du zinc, du cérium et du fer ainsi que les mélanges quelconques des composants mentionnés, qui peuvent être modifiés en surface.

10. Compositions selon l'une quelconque des revendications 1 à 9, **caractérisées en ce qu'**elles contiennent en outre au moins un polymère.

11. Compositions selon la revendication 10, **caractérisées en ce que** le polymère est choisi parmi les polymères anioniques et amphotères naturels et synthétiques ainsi que parmi les polymères non ioniques synthétiques.

12. Compositions selon l'une quelconque des revendications 1 à 11, **caractérisées en ce qu'**elles contiennent en outre au moins un co-émulsifiant non ionique du type des substances grasses polaires ou des émulsifiants eau-dans-huile présentant une valeur BHL inférieure à 6,5.

13. Compositions selon la revendication 12, **caractérisées en ce que** le co-émulsifiant présentant une valeur BHL inférieure à 6,5 est choisi parmi les alcools gras en C₁₂-C₂₂ linéaires et les esters partiels d'acides gras en C₁₂-C₂₂ de polyols en C₂-C₆.

14. Compositions selon la revendication 12 ou 13, **caractérisées en ce que** le rapport pondéral de co-émulsifiant à composant polaire huileux est de 0,1:1 à 1:1.

15. Compositions selon l'une quelconque des revendications 1 à 14, **caractérisées en ce qu'**elles contiennent en outre au moins une vitamine, une provitamine, un précurseur de vitamine ou leurs dérivés des groupes des vitamines A, B, C, E, F et H.
